# EUROPEAN PATENT APPLICATION

(11) **EP 4 732 799 A1**
(43) Date of publication of application: **29.04.2026**
(21) Application number: 24209255.9
(22) Date of filing: 28.10.2024
(51) Int. Cl.: A61B 18/14, A61B 1/00, A61B 90/00, A61B 1/307, A61B 17/00, A61B 18/00, A61B 34/20

(54) **IMAGING SUPPORT OF TRANSURETHRAL PROSTATE RESECTION AND TURP SYSTEM**

(71) Applicant: OLYMPUS WINTER & IBE GMBH, 22045 Hamburg (DE)
(72) Inventor: SUPPA, Per, 22083 Hamburg (DE)
(74) Representative: Seemann & Partner Patentanwälte mbB

(57) **Abstract**

A system for navigation and/or control support for transurethral prostate resection (TURP), comprising a controller configured to carry out the following steps:
a) analysing endoscopic images provided by a resectoscope (20) and/or additional sensory data from within the resectoscope (20) during TURP for one or more of several options, and at least one of
b) automatically disabling HF current to the resection electrode (24) under several different outcomes of analysing step a), and
c) outputting visual and/or acoustic information to an operator performing the TURP, the information comprising at least one of
i) a visual indication of an anatomical landmark inside and/or outside of the endoscopic image,
ii) a visual indication of a distance of the resection electrode (24) to an anatomical landmark, and
iii) a visual or acoustic warning signal when the resection electrode (24) comes closer than a predetermined allowable distance to one or more of the bladder neck (10), the verumontanum (10) and the internal and/or external sphincter muscles (6, 8).

## Description

The present application refers to a method for imaging support of transurethral prostate resection and a TURP system.

Benign Prostate Hyperplasia (BPH) leads to lower urinary tract symptoms and is common among male patients beyond 60 years of age. Transurethral resection of the prostate (TURP) is the gold standard treatment for BPH, especially for highly enlarged prostates. TURP is a surgical procedure, where a urologist inserts a resectoscope into the urethra and uses it to remove prostate tissue, which leads to a release of pressure on the urethra. The resectoscope is equipped with a resection electrode, most commonly in the form of a sling, and high frequency (HF) alternating current from an energy generator can be used to cut tissue (i.e., resect) of the prostate. The resection electrode is usually housed in an inner shaft of the resectoscope that can be moved forward and backward with respect to the outer shaft of the resectoscope, which also houses the optical system of the resectoscope.

The TURP procedure is associated with several postoperative complications, such as retrograde ejaculation, urethral strictures, urinary tract infection, bladder neck contractures, erectile dysfunction, postoperative bleeding and urinary retention. Of these, retrograde ejaculation is by far the most frequent complication affecting 65-100% of patients after TURP. Although retrograde ejaculation does not affect the ability to have an orgasm, it feels uncomfortable as semen goes into the bladder instead of out of the penis and hence affects the patient's quality of life. The primary cause for retrograde ejaculation is injury to the internal and/or external sphincter during TURP, both of which are responsible for maintaining continence of urine.

Performing TURP, the urologist faces a number of challenges in avoiding retrograde ejaculation and other complications, such as postoperative bleeding. Most complications arise from accidental tissue damage, such as damage of the external and/or the internal sphincter muscle, but also of other landmarks of the urinary tract. One example for this kind of damage is an inadvertent resection of the verumontanum. Also, if the electrode is accidentally activated while pushing the scope forward, this can cause tunnelling, perforation, bladder injury or extravasation, among others.

It is therefore an object of the present disclosure to provide aid to an urologist performing a transurethral resection of the prostate such as to avoid negative outcomes as described above.

This object is achieved by a method for navigation and/or control support for transurethral prostate resection (TURP), the method comprising the following steps:
a) analysing endoscopic images provided by a resectoscope and/or additional sensory data from within the resectoscope during TURP for one or more of
   i) detection of movement of the resectoscope in a forward or backward direction,
   ii) detection of movement of an inner shaft of the resectoscope housing a resection electrode in a forward or backward direction,
   iii) automatic detection of one or more anatomical landmarks of the urinary tract,
   iv) automatic tracking of the one or more anatomical landmarks of the urinary tract, and
   v) automatic detection of the resection electrode's position between bladder neck and verumontanum,
   and at least one of
b) automatically disabling HF current to the resection electrode when in the analysing step a), it is found that
   i) the resectoscope is moving in a forward direction,
   ii) the inner shaft of the resectoscope is moving in a forward direction,
   iii) the resection electrode comes closer than a predetermined allowable distance to one or more of the bladder neck, the verumontanum and the internal and/or external sphincter muscles,
   iv) wobbling of the resectoscope is detected, or
   v) patient movement is detected,
c) outputting visual and/or acoustic information to an operator performing the TURP, the information comprising at least one of
   i) a visual indication of an anatomical landmark inside and/or outside of the endoscopic image,
   ii) a visual indication of a distance of the resection electrode (24) to an anatomical landmark, and
   iii) a visual or acoustic warning signal when the resection electrode (24) comes closer than a predetermined allowable distance to one or more of the bladder neck, the verumontanum and the internal and/or external sphincter muscles.

The method provides the urologist performing TURP with enhanced navigation and/or control support by eliminating several common causes for intraoperative complications and providing the operator with added guidance for the task she has to perform, thus improving patient outcome over the unassisted case. In order to do this, step a) of image analysis is performed and in addition at least one of steps b) of disabling HF current and c) of issuing a warning under certain circumstances listed thereunder.

The method provides for, a. o., detecting from the resectoscope image if the resectoscope is moving in backward or forward direction. Based on this knowledge, the HF cutting current to the electrode can be automatically disabled according to method step b) i) when the resectoscope is moved forward in order to enable to provide cleanly separable slices of tissue, thus preventing tunneling, perforation, bladder injury and extravasation. It will furthermore, guarantee that the internal sphincter muscle located at the bladder neck will not be damaged due to HF current.

The method also provides for detection from the resectoscope image of whether the inner shaft with the electrode is moving backwards or forwards. In case of forward movement of the electrode (pushing), the HF cutting current is automatically disabled according to method step b) ii), thus avoiding any damage to anatomical structures, such as the verumontanum or the bladder neck.

The functionality of automatic detection of anatomical landmarks is useful in navigation and control. These landmarks, in particular, include the verumontanum and the bladder neck. The region between these two landmarks is important since they delimit the area between the internal and external sphincter muscles where the resection should take place. In order to prevent damage to the internal sphincter muscle, the electrode should not exceed the bladder neck during pushing. Furthermore, the electrode should not be pulled back further than the tip of the verumontanum in order to prevent damage to the external sphincter muscle, as the external sphincter muscle is located next to and behind this anatomical landmark.

In addition to their detection, the anatomical landmarks may also be automatically tracked. The automatic tracking is to be understood to mean that, once the landmarks have been detected, the position, preferably including distance and orientation, of the resection electrode with respect to the tracked landmarks, is tracked during the procedure, even when further movement of the resectoscope moves one or more of the landmarks out of the resectoscope's field of view. Such tracking is also useful in avoiding damage to the landmarks or, respectively, the internal and external sphincter muscles, when a curved resection at the lateral lobes of the prostate is performed, wherein the resection electrode is oriented in angled position. In this case, it is important for the operator to understand where resection is performed in relation to these landmarks, even if they are not visible at the moment. This understanding helps the operator avoid accidentally damaging the landmarks, including the ureters, and the lateral lobes of the prostate due to under- or overestimation of space for pulling/pushing the resection electrode during tissue resection.

Such tracking can also help detect "wobbling" of the resectoscope, i.e., changes in position in different directions or "jittering", caused by wobbling of the external sphincter. Being aware of such wobbling will assist in avoiding permanent sphincteric damage and incontinence. In the case of the detection of wobbling of the resectoscope, the HF current from the HF generator to the resection electrode may be blocked in order to avoid damage to any anatomical structures.

Since landmark tracking is connected to movements of the resectoscope, it has a similar functionality as the above-mentioned movement detection in the forward and backward direction, with an added component of determination of orientation.

An automatic determination of the electrode's position between the bladder neck and the verumontanum assists in, among others, avoiding inadvertent resection or cauterization of the verumontanum, finding out whether a resection has been done completely within a particular area, assessing the surgical margin when resecting lateral lobes, avoiding accidental resection of the ureter.

An automatic triggering of a warning signal or visualization on the screen when the tip of electrode comes too close to either the bladder neck or the verumontanum assists in preventing damage to the internal and/or external sphincter muscle, alerting the operator of the increased risk in this area. This can be accompanied by the blocking of the HF current from the HF generator under these circumstances.

The method in particular does not include any steps pertaining to the TURP procedure itself, but only those steps that pertain to analysing the endoscopic images and generating control signals to the HF generator and information to be displayed or sounded.

In embodiments, the detection of movement of the resectoscope in a forward or backward direction of method step a) i) is done using a machine learning (ML) algorithm, in particular an ML algorithm implementing pose estimation, or using optical flow technique.

In further embodiments, the detection of movement of an inner shaft of the resectoscope housing the resection electrode in a forward or backward direction of method step a) ii) is done using, in particular ML based, detection and tracking of a sling of the resection electrode in the endoscopic images. This makes use of the fact that the electrode in the image appears smaller when pushed towards the bladder neck and bigger when pulled back. Slings have a distinctive shape that differs in shape and colour from anatomical structures, which can be observed in conventional image processing. Alternatively, such distinctive structures are easily trained to be recognized by a CNN (convolutional neural network).

The automatic detection of one or more anatomical landmarks of the urinary tract of method step a) iii) and/or automatic detection of the resection electrode's position between bladder neck and verumontanum of method step a) v) is or are done using a trained ML algorithm, in particular a trained CNN, wherein in particular annotated training data from BPH videos are used to teach the ML algorithm to detect anatomical landmarks on video frames. The distance between the verumontanum, directly positioned under the resectoscope's outer shaft, and the bladder neck can be determined using ML-based monocular depth estimation.

Alternatively or additionally, if the resectoscope is equipped with sensors between the inner and the outer shaft that are sensitive to movements of the inner shaft with respect to the outer shaft, sensor signals from those sensors can be used for the same purpose. Based on the sensor signals, it can be determined whether there is a forward or backward motion of the resection electrode with respect to the resectoscope, or how far the resection electrode has been extended from the tip of the outer shaft of the resectoscope, or whether there is a torque providing a sideways pressure on the resection electrode with respect to the outer shaft. Appropriate sensors may be, but not limited to, strain gauges attached to the inner shaft in equidistant positions, combined with elevations, such as blister shaped, bubble shaped or spiked elevations, attached to the outer shaft, or vice versa, which are arranged such relative to the strain gauges as to contact and deflect them when the inner and outer shafts are moved relative to each other, so that each deflection or firing of the strain gauge sensors allows the system to understand the direction and amount of movement of the inner shaft and hence the rejection electrode.

The distance between the verumontanum and the bladder neck can be determined with both approaches, that is, either by monocular depth estimation and by the strain gauge sensor technique, or both in combination with each other, by pushing out the inner shaft with the resection electrode as close as possible to the bladder neck from the initial position close to the verumontanum and measuring the number of deflections or firings of the strain gauge sensors to determine the distance between both anatomical landmarks. The elevations and strain gauge sensors are attached in a way that they cannot only deflect forward and backward movements that also movements in all directions.

The distance as measured with either of these options or a combination thereof will then be stored by the system, that is, registered. Together with the detection of movement of the resectoscope, significant movement of the resectoscope in relation to the verumontanum can be detected. In this case, the user of the system will be alerted about the change by displaying of a warning message on the screen, for example, and will be prompted to reposition the resectoscope to its optimal position in relation to the verumontanum.

In embodiments, the analysis of endoscopic images in method step a) iv) is done using a ML-based CNN tracking algorithm, such as described in M. S. Bahraini et al., "SLAM in Dynamic Environments: A Deep Learning Approach for Moving Object Tracking using ML-RAN-SAC Algorithm.", Sensors 2019, 19(17), 3699. As soon as an anatomical landmark is not visible in the resectoscope image any more, relative tracking based on the last visible position of this landmark in the image and the determination of resectoscope movement as described above can be used to track the position of the anatomical landmark even if it is not visible in the image any more.

In further embodiments, the automatic detection of the resection electrode's position between bladder neck and verumontanum of method step a) v) is done using the sensors between the inner and outer shafts of the resectoscope, i.e., e.g., the above described strain gauge sensors and elevations.

Both the above-described, in particular ML-based, image-based motion detection as well as the sensors between the inner shaft and the outer shaft of the resectoscope make it possible, each on its own or in combination, to detect wobbling of the resectoscope. In embodiments, a threshold can be defined to define whether observed motion is significant wobbling, in order to differentiate it from tremors of the resectoscope holder.

The previously described features also make it possible to put into practice the determination of a distance between the bladder neck and the verumontanum in combination with the determination of the resection electrode's position between these two landmarks. If the position of the resection electrode is detected as being too close to any of the anatomical landmarks, which have to be preserved, a warning message can be displayed on a screen, a warning sound can be played, and/or the HF current to the resection electrode can be automatically turned off, according to method steps b) iii) and/or c) iii).

The object of the present disclosure is also achieved by a transurethral prostate resection system, comprising a resectoscope with a resection electrode and an optical imager, a HF generator connected to provide HF current to the resection electrode and a controller configured to control the generation of HF current by the HF generator and to perform image processing on endoscopic video images provided by the optical imager of the resectoscope, wherein the controller is configured to carry out the above described method for navigation and/or control support for transurethral prostate resection (TURP) according to the present disclosure.

The object of the present disclosure is also achieved by a software program with program code and/or a computer readable medium storing such software program, the program being configured to carry out the above described method for navigation and/or control support for transurethral prostate resection (TURP) when loaded into the memory of and run on the controller of the above described transurethral prostate resection system.

Furthermore, the object may be achieved by a method of performing a transurethral resection procedure using the above described method for navigation and/or control support and/or the above described system.

The features, advantages and characteristics of the above described method thereby are also incorporated in the system, the software program and the computer readable medium.

Further characteristics of the invention will become apparent from the description of the embodiments according to the invention together with the claims and the included drawings. Embodiments according to the invention can fulfil individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a representation of portions of the male urinary tract,
- Fig. 2: a representation of an endoscopic image during a TURP procedure,
- Fig. 3: a schematic representation of a resectoscope and
- Fig. 4: two schematic representations of resectoscope actions.

In the drawings, the same or similar types of elements or respectively corresponding parts are provided with the same reference numbers in order to prevent the item from needing to be reintroduced.

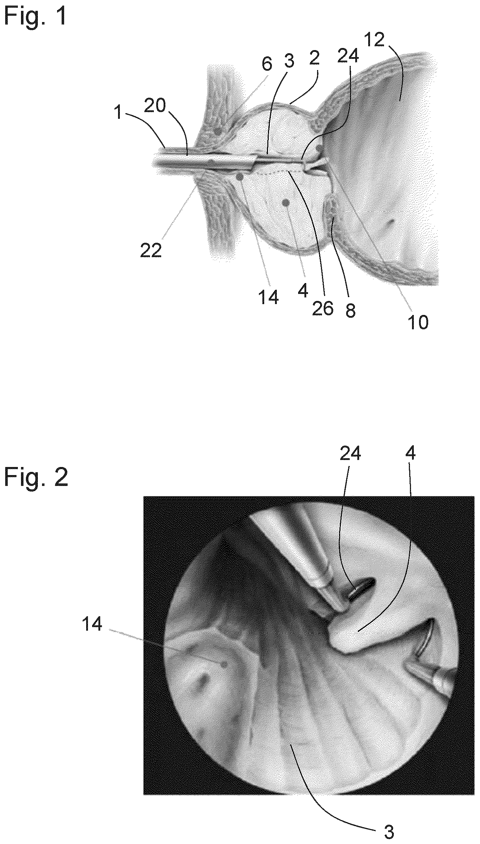

Fig. 1 illustrates a representation of portions of the male urinary tract during a transurethral resection of the prostate (TURP) procedure. On the left, the external urethral sphincter 6 is shown, a muscle that clamps down on the urethra 1. It is followed by the prostate 2, the internal urethral sphincter 8 and the bladder 12. The bladder 12 is separated from the prostate 2 by the bladder neck 10.

Inside the prostate 2, there is prostate tissue 4 surrounding the prostatic urethra 3, some of which is to be resected in order to lower the pressure the prostate tissue 4 applies to the prostatic urethra 3.

To this end, a resectoscope 20, whose outer shaft 22 is shown in Fig. 1, is inserted through the urethra 1 into the prostatic urethra 3. It is equipped with a resection electrode 24, most commonly in the shape of a sling, that is fed with a high frequency (HF) current for cutting prostate tissue 4. In Fig. 1, the resection electrode 24 is extended from the outer shaft 22 of the resectoscope, to be pulled back towards the outer shaft 22 for a cutting action. A resection plane 26 of the next cut is overlaid as a dotted line.

Inside the prostate, the outer shaft 22 of the resectoscope 20 is positioned on top of a landmark of the prostate 2, namely the verumontanum 14. It, as well as the internal and external urethral sphincters 8, 6, and the bladder neck 10, should ideally left intact during TURP. However, unaided, there is risk that HF current is applied to resection electrode 24 during forward motion or during involuntary motions, thus potentially causing damage to surrounding tissues and landmarks, including punching through to the bladder 12, giving rise to unwanted retrograde ejaculation.

Fig. 2 represents an endoscopic image during a TURP procedure without any additional information. The optical system of the resectoscope includes lighting and imaging functionality. Its interface to the operation area is located at the tip of the outer shaft 22 of the resectoscope, over which the resection electrode 24 extends. The endoscopic image therefore shows the sling shaped electrode 24 inside the prostatic urethra 3, which has already been partially resected, as can be seen from the plurality of parallel furrows running along the prostatic urethra 3. The furrows stop short of the verumontanum 14 in the lower left corner, which has been left intact. The resection continues in the upper right part of the prostatic urethra 3 opposite the verumontanum 14.

The endoscopic images produced by the resectoscope 20 are subjected to the above described image processing according to the present disclosure for the purpose of automatically disabling HF current to the resection electrode 24 under the above described circumstances, and/or for the purpose of generating information and/or warnings for the operator as described above.

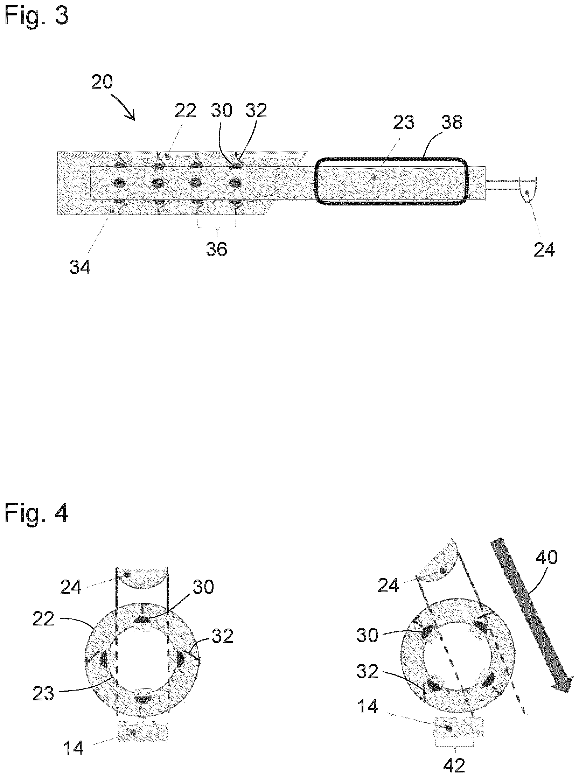

Fig. 3 is a schematic partial representation of an embodiment of a resectoscope 20 that is usable in the presently disclosed method and system. This illustration highlights a suite of sensors to be placed in the region 38 near the distal end of the outer shaft 22, whereas the sensors themselves are shown in more detail in the left part of Fig. 3. The sensors comprise elevations 30 placed on the outside of the inner shaft 23 in an equidistant spacing 36, possible in several rows. In opposition facing the elevations 30, strain gauge sensors 32 are placed, also equidistantly, on the inside of the outer shaft 22 of resectoscope 20, such as to interact with the elevations 30 when the inner shaft 23 is moved with respect to the outer shaft 22.

In Fig. 3, it is shown that the inner shaft 23 is pushed forward (i.e., to the right) with respect to the outer shaft. Through this motion, there is interaction 34 between the elevations 30 and the strain gauge sensors 32, wherein the elevations 30, being pushed forward along with the inner shaft 23 they are attached to, contact the strain gauge sensors 32 and bend them forward. If the strain gauge sensors 32 are sensitive to the direction of bending, the direction of the movement is immediately discernible. Furthermore, if the forward movement of the inner shaft 23 with respect to the outer shaft 22 continues on, each strain gauge sensor 32 will send out repeated signals as each of a number of elevations 30 brushes past the respective strain gauge sensors 32. Since the distance between the elevations 30 and the strain gauge sensors 32 are known, the number of sensor signals provides an accurate measure of the distance the resection electrode 24 has been pushed forward out of the outer shaft 22 of the resectoscope 20.

Fig. 4 two schematic cross section views through the resectoscope 20 of Fig. 3. The images illustrate that the elevations 30 and strain gauge sensors 32 can also be used to track the rotation of the inner shaft 23 of the resectoscope 20 with respect to the outer shaft 22. The resectoscope 20 is positioned over the verumontanum 14. The orientation of the resection electrode 14 is visible at the top. When pulled back along the parallel dotted lines, its orientation does not change.

On the right of Fig. 4, there is an example of a rotated inner tube. The rotation angle can be determined, and based on the rotation angle, the amount of movement 42 can be determined, which allows the determination of the location of the verumontanum 14 in relation to the resection electrode 24 in order to avoid damage to this anatomical landmark through pulling of the resection electrode 14 in pulling direction 40 during resection.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 1: urethra
- 2: prostate
- 3: prostatic urethra
- 4: prostate tissue to resect
- 6: external urethral sphincter
- 8: internal urethral sphincter
- 10: bladder neck
- 12: bladder
- 14: verumontanum
- 20: resectoscope
- 22: outer shaft
- 23: inner shaft
- 24: resection electrode
- 26: resection plane of next cut
- 30: bubble shaped elevation
- 32: strain gauge sensor
- 34: sensor interaction
- 36: equidistant spacing
- 38: region for placement of sensors
- 40: pulling direction
- 42: amount of movement

## Claims

1. Method for navigation and/or control support for transurethral prostate resection (TURP), the method comprising the following steps:
a) analysing endoscopic images provided by a resectoscope (20) and/or additional sensory data from within the resectoscope (20) during TURP for one or more of
i) detection of movement of the resectoscope (20) in a forward or backward direction,
ii) detection of movement of an inner shaft (23) of the resectoscope (20) housing a resection electrode (24) in a forward or backward direction,
iii) automatic detection of one or more anatomical landmarks of the urinary tract,
iv) automatic tracking of the one or more anatomical landmarks of the urinary tract, and
v) automatic detection of the resection electrode's (23) position between bladder neck (10) and verumontanum (14),
and at least one of
b) automatically disabling HF current to the resection electrode (24) when in the analysing step a), it is found that
i) the resectoscope (20) is moving in a forward direction,
ii) the inner shaft (23) of the resectoscope (20) is moving in a forward direction,
iii) the resection electrode (24) comes closer than a predetermined allowable distance to one or more of the bladder neck (10), the verumontanum (14) and the internal and/or external sphincter muscles (6, 8),
iv) wobbling of the resectoscope (20) is detected, or
v) patient movement is detected,
and
c) outputting visual and/or acoustic information to an operator performing the TURP, the information comprising at least one of
i) a visual indication of an anatomical landmark inside and/or outside of the endoscopic image,
ii) a visual indication of a distance of the resection electrode (24) to an anatomical landmark, and
iii) a visual or acoustic warning signal when the resection electrode (24) comes closer than a predetermined allowable distance to one or more of the bladder neck (10), the verumontanum (10) and the internal and/or external sphincter muscles (6, 8).

2. The method according to claim 1, **characterized in that** the detection of movement of the resectoscope (20) in a forward or backward direction of method step a) i) is done using a machine learning (ML) algorithm, in particular an ML algorithm implementing pose estimation, or using optical flow technique.

3. The method according to claim 1 or 2, **characterized in that** the detection of movement of an inner shaft (23) of the resectoscope (20) housing the resection electrode (24) in a forward or backward direction of method step a) ii) is done using, in particular ML based, detection and tracking of a sling of the resection electrode (24) in the endoscopic images.

4. The method according to one of claims 1 to 3, **characterized in that** the automatic detection of one or more anatomical landmarks of the urinary tract of method step a) iii) and/or automatic detection of the resection electrode's (23) position between bladder neck (10) and verumontanum (14) of method step a) v) is or are done using a trained ML algorithm, in particular a trained CNN, wherein in particular annotated training data from BPH videos are used to teach the ML algorithm to detect anatomical landmarks on video frames.

5. The method according to one of claims 1 to 4, **characterized in that** a distance between the verumontanum (14) and the bladder neck (10) is determined using ML-based monocular depth estimation.

6. The method according to one of claims 1 to 5, **characterized in that** the resectoscope (20) is equipped with sensors between its inner shaft (23) and its outer shaft (22) that are sensitive to movements of the inner shaft (23) with respect to the outer shaft (22), wherein sensor signals from those sensors are used for motion detection of the inner shaft (23) relative to the outer shaft (22).

7. The method according to one of claims 1 to 6, **characterized in that** the analysis of endoscopic images in method step a) iv) is done using a ML-based CNN tracking algorithm.

8. The method according to of claims 4 and 6, **characterized in that** the automatic detection of the resection electrode's (24) position between bladder neck (10) and verumontanum (14) of method step a) v) is done using the sensors between the inner and outer shafts (22, 23) of the resectoscope (20).

9. Transurethral prostate resection system, comprising a resectoscope (20) with a resection electrode (24) and an optical imager, a HF generator connected to provide HF current to the resection electrode (24) and a controller configured to control the generation of HF current by the HF generator and to perform image processing on endoscopic video images provided by the optical imager of the resectoscope (20), **characterized in that** the controller is configured to carry out a method for navigation and/or control support for transurethral prostate resection (TURP) according to one of claims 1 to 8.

10. Software program with program code, configured to carry out the method for navigation and/or control support for transurethral prostate resection (TURP) according to one of claims 1 to 8 when loaded into the memory of and run on the controller of the transurethral prostate resection system according to claim 9.
